Europäisches Patentamt

European Patent Office

Office européen des brevets

(11) Numéro de publication : **0 537 092 A1**

(12) # DEMANDE DE BREVET EUROPEEN

(21) Numéro de dépôt : **92420355.7**

(22) Date de dépôt : **08.10.92**

(51) Int. Cl.[5] : **A61K 9/127,** A61K 7/00, C12N 5/00

(30) Priorité : **08.10.91 FR 9112597**
**08.10.91 FR 9112598**
**08.10.91 FR 9112599**

(43) Date de publication de la demande :
**14.04.93 Bulletin 93/15**

(84) Etats contractants désignés :
**AT BE CH DE DK ES FR GB GR IE IT LI LU MC NL PT SE**

(71) Demandeur : **PATRINOVE SOCIETE CIVILE**
**39, rue du Lieutenant-Colonel-Prévost**
**F-69006 Lyon (FR)**

(71) Demandeur : **TEXINFINE SOCIETE ANONYME**
**60, rue Duguesclin**
**F-69006 Lyon (FR)**

(71) Demandeur : **SOCIETE D'HEPATOLOGIE EXPERIMENTALE**
**Avenue Tony Garnier**
**F-69007 Lyon (FR)**

(72) Inventeur : **Gutierrez, Gilles**
**39 Rue Lieutenant Colonel Prévost**
**F-69006 Lyon (FR)**
Inventeur : **Grimaud, Jean-Alexis**
**48 Chemin de la Gloire de Dieu**
**F-38200 Vienne (FR)**
Inventeur : **Andujar, Mauricio**
**9 Rue Laurent Vibert**
**F-69006 Lyon (FR)**
Inventeur : **Serrar, Mostapha**
**81 Rue du 1er Mars**
**F-69100 Villeurbanne (FR)**
Inventeur : **Emonard, Hervé**
**113 Avenue Elizabeth**
**B-4802 Heusy (BE)**
Inventeur : **Melin, Martine**
**5 Rue de Cuzieu**
**F-69110 Saint Foy Les Lyon (FR)**

(74) Mandataire : **Monnier, Guy et al**
**Cabinet Monnier 150 Cours Lafayette B.P. 3058**
**F-69393 Lyon Cédex 03 (FR)**

(54) **Vecteurs vésiculaires infracellulaires et leurs applications dans les compositions cosmétiques, médicamenteuses et les milieux de culture.**

(57)    Ces vecteurs vésiculaires infracellulaires qui comportent des ligands métaboliques aptes à s'exprimer à l'intérieur et à l'extérieur de leur structure, sont d'affinité spécifique du fibroblaste dont la composition membranaire, modifiée par introduction de ligands métaboliques, entraîne une modulation de l'activité de synthèse de la cellule ciblée.

Ils sont choisis parmi les vésicules, capsules ou sphères artificielles d'un diamètre qui peut aller de 30 à 1000 nanomètres, mis en oeuvre pour incorporer une substance active soit dans un milieu aqueux remplissant le volume intérieur du vecteur soit dans la paroi même dudit vecteur puis pour transporter cette substance active vers un site où elle sera libérée et utilisée à différentes fins.

Les actifs sont encapsulés à des concentrations variant de 0,01 % à 2 % ($10^{-5}$) $10^{-2}$) par rapport à la phase lipidique totale.

Les ligands métaboliques sont choisis parmi les initiateurs (esters gras de l'acide ascorbique) et les modulateurs de la synthèse cellulaire (palmitate d'hydrolysat de collagène, esters gras de l'hydroxyproline)dotés de fonction ou radical lipophile par acylation.

Ils trouvent des applications dans les compositions cosmétiques destinées au raffermissement du tissu cutané, au traitement des petites cicatrices indurées et des ridules, et à provoquer un renforcement de la couche épithéliale ainsi que dans les compositions médicamenteuses destinées à l'augmentation de la synthèse des collagénases en vue de résorber les tissus fibreux denses en collagène I ou les chéloïdes, ainsi qu'à l'orientation de la synthèse du collagène vers une structure particulière telle que les collagènes I et III.

Enfin ils s'appliquent à la réalisation de milieux de culture pour la production de peptides de la matrice extracellulaire et pour la production de peptides à activité enzymatique.

EP 0 537 092 A1

La présente invention concerne des vecteurs vésiculaires infracellulaires , ainsi que leurs applications dans des compositions cosmétiques, des compositions médicamenteuses destinées à favoriser le renouvellement de la matrice extracellulaire et des milieux synthétiques pour la culture des fibroblastes.

On sait que l'abondance de la matrice extracellulaire qui sépare les constituants cellulaires est l'une des caractéristiques du tissu conjonctif. Cette matrice est à l'origine de différentes fonctions remplies par ce tissu. Elle sert d'élément de support biomécanique et sa composition variée influence le comportement cellulaire.

Loin d'être une structure stable, cette matrice est en remaniement constant du fait de l'activité cellulaire qui la synthétise et la modifie. Les changements se produisant au niveau de la matrice extracellulaire peuvent être de nature quantitative ou de nature qualitative. Un déséquilibre entre la synthèse et/ou la dégradation peut contribuer à la mise en place d'une matrice extracellulaire "modifiée" ou pathologique.

Une très grande variété des molécules qui composent la matrice extracellulaire (collagènes, protéoglycanes et glycoprotéines) sont par ailleurs synthétisées par les fibroblastes.

Le collagène représente un des composants majeurs de la matrice extracellulaire. On connaît actuellement 15 types de collagènes originaires d'une famille d'une vingtaine de gênes. Ces molécules sont formées d'une association de trois chaînes peptidiques en triple hélice alpha. Dans la partie hélicoïdale, une séquence d'amino-acide se répète, où la glycine précède souvent une proline et une hydroxyproline. La structure protéique spécifique permet aux collagènes d'exercer différentes fonctions au sein du tissu conjonctif.

Parmi les différents types de collagène, les collagènes de type I et III constituent le produit de synthèse majeur des fibroblastes. Emonard et Grimaud (Cellular and Molecular Biology, 36 (2) 131-153 - 1990) ont montré que les fibroblastes peuvent contrôler la qualité et la quantité des collagènes non seulement par la diminution de la synthèse, mais aussi par la synthèse des enzymes de dégradation correspondants. Ainsi, la cellule fibroblastique peut, à elle seule, contrôler l'ensemble du dépôt matriciel conjonctif. Cette cellule fibroblastique est de plus influencée en permanence par des facteurs solubles (cytokines) et par des facteurs insolubles (les composants matriciels).

Le résultat de ces interactions conditionne la fonction fibroblastique vers une activité de synthèse ou de dégradation à l'égard de la matrice extracellulaire.

Les fibroblastes constituent donc une pièce fondamentale dans la physiologie du tissu conjonctif et le contrôle de ses fonctions est d'une importance capitale tant dans le domaine cosmétique que dans le domaine médical.

Le contrôle de la synthèse du collagène par les fibroblastes est d'une importance majeure pour la physiologie du tissu conjonctif. En effet, le fibroblaste est la cellule mésenchymateuse responsable de la synthèse, de l'organisation et du remodelage de la matrice conjonctive. Parmi les différentes molécules synthétisées par les fibroblastes, nous avons vu que les collagènes de type I et III sont quantitativement les plus importants.

De ce fait, l'utilisation de la culture de fibroblastes permet d'analyser de manière précise l'action des différentes molécules sur l'activité de synthèse ou de dégradation du collagène.

Un grand nombre de drogues ou molécules ont déjà été décrites comme susceptibles de modifier la fonction fibroblastique (Bissel et Coll, Hepatology ,vol. II n° 3,1990 p. 488-498 - Emonard et Grimaud, in Cellular and Molecular Biology, 36 (2) 131-153 - 1990 - Kovacs in Immunology Today Vol. 12, N 1, 1991).

Certaines de celles-ci sont capables de stimuler la synthèse du collagène par les fibroblastes : l'acide ascorbique (Chojkier et coll - Jal of Biological Chemistry - Oct. 5,1989 p. 16957 - 16962), les cytokines comme l'interleukine-1, le facteur de croissance dérivé des plaquettes (PDGF) (Kovacs), les peptides dérivés du collagène (Pacini et coll. Res. Comm. in Chemical Pathology and Pharmacology - Avril 1990 p. 89-101).

D'autres molécules ont été étudiées pour inhiber la synthèse du collagène et parfois induire la synthèse d'enzymes de dégradation (collagénases). Les plus connues sont les hormones corticoïdes, les cytokines comme le facteur dérivé de la nécrose tumorale (TNF) et les interférons ; voir : Bissel et Coll , Emonard et Grimaud, Granstein et coll. (The Jal of Investigative Dermatology suppl. déc. 1990) et Kovacs.

On a toutefois constaté que les milieux nutritifs les meilleurs ne permettaient pas d'augmenter la teneur en acides aminés au sein des cytoplasmes cellulaires.

Or on sait que la synthèse du collagène nécessite un apport important en acides aminés.

Il n'est notamment pas possible d'accroître de manière significative la teneur en acide ascorbique d'un milieu nutritif en raison du pH ou des modifications de la pression osmotique lorsque celui-ci est neutralisé.

Les vecteurs vésiculaires infracellulaires selon l'invention, qui comportent des ligands métaboliques aptes à s'exprimer à l'intérieur et à l'extérieur de leur structure, sont caractérisés en ce qu'ils sont des vecteurs d'affinité spécifique du fibroblaste dont la composition membranaire, modifiée par introduction de ligands métaboliques hydrophiles, entraîne une modulation de l'activité de synthèse de la cellule ciblée.

Les inventeurs ont constaté de façon surprenante que l'utilisation de ces vecteurs vésiculaires infracellulaires permettait d'ajouter au milieu de culture des éléments nutritifs n'intervenant ni dans le pH ni dans l'expression de la pression osmotique.

Dans la présente description, le terme vecteur vésiculaire (ou pour simplifier "vecteurs") s'appliquera à tous vésicules, capsules ou sphères artificielles d'un diamètre qui peut aller de 30 à 1000 nanomètres, et qui sont mis en oeuvre pour incorporer une substance active soit dans un milieu aqueux remplissant le volume intérieur du vecteur soit dans la paroi même dudit vecteur puis pour transporter cette substance active vers un site où elle sera libérée et utilisée à différentes fins.

Les vecteurs vésiculaires selon l'invention sont de préférence obtenus par mise en oeuvre du procédé et du dispositif décrits dans FR - A - 89 09 275 du 5 Juillet 1989

Ce procédé repose sur l'utilisation d'un phénomène très particulier que l'on pourrait appeler "coup de bélier". Ce phénomène apparaît lorsqu'une veine liquide s'écoule très rapidement au sein d'un conduit rigide. L'écoulement rapide engendre des zones de surpressions et des zones de dépressions très intenses.

Les zones de dépressions conrrespondent à des zones de cavitation. Ce phénomène apparaît aussi de manière ponctuelle et transitoire lorsque le fluide subit de brutales variations de débit. Dans ce cas, l'écoulement n'étant pas continu, il n'est pas possible d'entretenir le phénomène de cavitation et, quand un solide se déplace à très grande vitesse au sein d'un liquide, on ne dispose pas de zones d'hyperpression.La succession de zones d'hyperpression et de zones de cavitation au sein de la veine liquide permet la synthèse des membranes vectoriales sans avoir recours à l'utilisation de solvant organique ou de chauffage pour inclure des substances lipidiques ou insolubles.

En effet, les zones d'hyperpression permettent de réaliser la fonte du matériel membranaire, d'une façon un peu similaire à celle utilisée lors de la formation des pastilles pour l'observation infrarouge. Les zones de cavitation assurent la sphérisation du matériel membranaire autour des bulles de gaz qui apparaissent au sein du liquide. On peut ainsi arriver à des taux d'encapsulation très élevés car les composés à encapsuler pourront servir de germes à l'apparition des bulles de gaz et seront donc préférentiellement insérés dans la vacuité vectoriale.

Les produits actifs peuvent être encapsulés dans des liposomes de structure lamellaire, à des concentrations variant de 0,01 % à 2 % ($10^{-5}$ à $10^{-2}$) par rapport à la phase lipidique totale.

On peut indiquer, à titre d'exemple:

| - Palminate de collagène : | 0,5 % |
|---|---|
| - Lécithine de soja hydrogénée (indice d'iode < 0,2) : | 7 % |
| - Cholestérol : | 0,01 % |
| - Tocophérols : | 0,02 % |
| - Ascorbate de soude : | 0,05 % |
| - Eau manitolée : | 5 % q.s. 100 ml |

Les formes liposomiques peuvent supporter une couche de Stern qui permet de porter plus loin le potentiel zeta de la vésicule. Cette couche de Stern est constituée de molécules hydrophiles fixées sur la partie extérieure de la vésicule.

Les actifs peuvent aussi être encapsulés dans d'autres formes vésiculaires telles que les nanocapsules, vésicules de constitution pariétale : palmitate de saccharose (5 %), cholestérol (5 %).

On peut enfin utiliser d'autres formes vectorisées et notamment les nanosphères à base de cire, de dérivés du siloxane, et des systèmes micellaires constitués par une couche d'ester ou d'amide d'un compose lipophile et d'un composé hydrophile.

Selon un mode de réalisation préféré de l'invention, les ligands métaboliques sont choisis parmi les initiateurs et les modulateurs de la synthèse cellulaire dotés de fonction ou radical lipophile par acylation, tels que les esters gras de l'acide ascorbique et notamment son palmitate, le palmitate d'hydrolysat de collagène, et les esters gras de l'hydroxyproline et plus spécialement son palmitate.

L'acide ascorbique est notamment immobilisé au sein du vecteur sous forme d"ester d'acide gras, ce qui évite toute fuite de composé.

L'hydrophilie de la membrane du vecteur est obtenue par la fixation d'amide grasse (lipoaminoacide) à l'extérieur du vecteur.

On voit tout l'intérêt du transport par un même vecteur d'un stimulant de la synthèse du collagène modifié pour une meilleure encapsulation et d'éléments précurseurs de la synthèse du collagène.

Selon l'invention l'utilisation des vecteurs vésiculaires cités plus haut en tant que vecteurs de molécules bioactives aptes à stimuler ou à moduler la synthèse des collagènes par les fibroblastes ou à inhiber cette synthèse et à induire la synthèse d'enzymes de dégradation renforce ou modifie considérablement l'action de ces molécules ; ils agissent de plus comme modificateurs de leur cytotoxicité ; ceci présente un intérêt considérable notamment dans l'application de ces vecteurs dans les domaines cosmétique et médicaux.

Les inventeurs ont découvert que ces vecteurs vésiculaires trouvaient des applications nouvelles et originales dans le contrôle de la synthèse du collagène par les fibroblastes.

Ces vecteurs vésiculaires trouvent des applications comme vecteurs de compositions cosmétiques destinées à obtenir un meilleur raffermissement du tissu cutané et notamment comme vecteurs de compositions cosmétiques destinées au traitement des petites cicatrices indurées et des ridules, ainsi que comme vecteurs de compositions cosmétiques destinées à provoquer un renforcement de la couche épithéliale.

Les inventeurs ont pu également déterminer que les aptitudes que présentent l'ensemble de ces vecteurs vésiculaires dans le contrôle de la synthèse du collagène par les fibroblastes leur permettaient de trouver des applications spécialement intéressantes dans la réalisation de compositions médicamenteuses destinées à favoriser le renouvellement de la matrice extra-cellulaire.

Les inventeurs ont pu enfin déterminer que les aptitudes que présentent l'ensemble de ces vecteurs vésiculaires dans le contrôle de la synthèse du collagène par les fibroblastes leur permettaient de trouver des applications spécialement intéressantes dans la réalisation de milieux de synthèse et de culture des fibroblastes.

En effet l'utilisation de ces vecteurs vésiculaires infracellulaires permet d'ajouter au milieu de culture des éléments nutritifs n'intervenant ni dans le pH ni dans l'expression de la pression osmotique.

Dans les expériences décrites ci-après on a utilisé plus spécifiquement le palmitate d'ascorbyle, des peptides dérivés du collagène, le palmitate d'hydroxyproline pour stimuler la synthèse du collagène ou de la collagénase.

Les résultats des essais présentés ci-après ont été obtenus selon la méthodologie suivante :

Conditions de culture : les fibroblastes sont obtenus à partir d'explants de peau humaine. Les cellules sont maintenues en culture dans du milieu DMEM (Gibco) additionné de 10 % de sérum de veau foetal (Gibco) et d'antibiotiques (pénicilline 100 U/ml, streptomycine 100 µg/ml).

Les subcultures sont réalisées par trypsination (Trypsin-EDTA, Gibco). Les cellules sont utilisées du 2e au 10e passage.

Conditions de traitements : Les cultures de fibroblastes témoins se réferent aux cultures avec vecteurs sans principe actif, en quantité similaire aux autres traitements. Les cultures traitées avec la molécule en solution se réferent aux conditions de la culture témoin et de la molécule sous forme soluble.

Les cultures traitées avec la forme vectoriale de la molécule impliquent que la molécule active soit inclue préalablement dans les vecteurs.

Test de prolifération cellulaire (test MTT) : après ensemencement, les cellules sont incubées trois jours avec du milieu contenant les molécules à tester. Puis on ajoute le sel de tétrazolium jaune (5 mg / ml). Il est réduit par les réductases mitochondriales des cellules vivantes en un produit formazan violet qui est ensuite solubilisé par de l'HCl 0,04 N dans de l'isopropanol.

La réaction colorée est lue à une longueur d'onde de 570 nm par un Multiskan Titertek (Flow Laboratories) et exprimée en densité optique.

Evaluation des collagènes solubles par dosage radioimmunologique : On prélève 100 µl de surnageant de culture et on y ajoute 100 µl d'anticorps anticollagène de type I ou anti-collagène de type III et 200 µl de PBS. Après 24 heures à 4°C, on ajoute 100 µl de collagène de type I ou de type III marqué à l'iode 125 ; on sépare ensuite de l'antigène libre l'antigène lié à l'anticorps par précipitation avec 100 µl d'antisérum de chèvre anti-IgG de lapin en présence de polyéthylène glycol (PEG) 6000. Après centrifugation on mesure la radioactivité du précipité à l'aide d'un compteur LKB 1260 multigamma.

Mesure de l'activité collagénasique dans le milieu de culture

On mesure la collagénase interstitielle qui dégrade les collagènes interstitiels natifs de type I-III.

a) Préparation du substrat : le collagène de type I, marqué au carbone-14, en solution dans $CH_3COOH$ 0,1 M est neutralisé à pH 7,4 par du Tris 1 M et dilué avec le tampon NaCl 0,15 M/$CaCl_2$, 4 mM/Tris-HCl 50 mM, pH 7,4 contenant du NEM 0,5 mM et du PMSF 0,1mM à raison de 1000cpm/100µl substrat (= 10000 cpm/essai).

b) Dosage de la collagénase: 100 µl de milieu sont activés par 10 µl de trypsine (1 mg/ml) à 37°C pendant 10 mn, suivi de l'addition de 10 µl de SBTI (5 mg/ml): 10 mn à température ambiante. 100 µl de milieu sont aussi testés sans activation par la trypsine (mesure de la forme active de l'enzyme).

Le substrat (10000 cpm/essai) est ajouté, éventuellement en présence d'EDTA 25 mM, le volume final étant de 250 µl. La réaction se déroule pendant 18 h à 25°C puis est stoppée par addition de 20 µl d'EDTA 250 mM (10 mn à température ambiante).Le mélange est ensuite chauffé 10 mn à 39°C.

Le collagène non dégradé est précipité par l'éthanol à la concentration finale de 18 % (30 mn à température ambiante).

Finalement, le mélange est centrifugé à 6000 g pendant 30 mn à 4°C. Des aliquots de 200 µl des surnageants sont mélangés avec 5 ml de liquide scintillant aqueux puis comptés pour leur radioactivité.

Une unité de collagénase est définie comme la quantité d'enzyme nécessaire pour dégrader 1 µg de collagène natif par heure à 25°C.

L'étude de l'influence des vecteurs vésiculaires infracellulaires selon l'invention va maintenant être décrite en détail pour chaque molécule.

PALMITATE D'ASCORBYLE

L'incorporation d'acide ascorbique sous forme de palmitate dans les vecteurs potentialise son action sur la synthèse de collagène par les fibroblastes.

On a pu en effet déterminer que la moitié de la dose de palmitate d'ascorbyle (12,5 µg/ml) incorporé aux vecteurs selon l'invention est capable d'induire le même niveau de synthèse de collagène de type I que le double de sa dose (25µg/ml) sous forme soluble. L'effet du palmitate d'ascorbyle incorporé aux vecteurs selon l'invention est encore plus forte sur la synthèse de collagène de type III. Les résultats sont rassemblés dans le tableau 1 ci-après.

## TABLEAU 1

### Stimulation de la synthèse de collagène par le palmitate d'ascorbyle après 48 heures de culture

|  | Collagène I ng/m | Collagène III ng/m |
|---|---|---|
| fibroblastes + vecteurs | 67 ± 13 | < 20 |
| fibroblastes + palmitate d'ascorbyle en solution : 12,5 µg/ml | 668 [a, b] ± 71 | 468 [a,b] ± 41 |
| fibroblastes + vecteurs chargés en palmitate d'ascorbyle 12,5 µg/ml | 950 [a,b] ± 58 | 882 [a,b] ± 64 |
| fibroblastes + palmitate d'ascorbyle en solution 25 µg/ml | 952 [a,b] ± 81 | 781 [a,b] ± 55 |
| fibroblastes + vecteurs chargés en palmitate d'ascorbyle 25 µg/l | 1275 [a,b] ±114 | 1035 [a,b] ± 95 |

[a] Significatif (p < 0,05) par rapport aux fibroblastes + vecteurs (contrôle)

[b] Significatif (p < 0,05) par rapport à la molécule soluble.

Ces résultats laissent apparaître tout l'intérêt de l'utilisation de ces vecteurs vésiculaires infracellulaires pour la préparation de produits cosmétiques visant à stimuler la synthèse de collagène afin d'obtenir un meilleur raffermissement de la peau, ainsi que pour la préparation de compositions médicamenteuses destinées à favoriser le dépôt d'une matrice extracellulaire nouvelle par un effet stimulant de la synthèse du collagène. On remarque également que le produit en phase vectoriale permet, pour une quantité plus faible de principe actif, d'obtenir un effet similaire.

Par ailleurs, ces vecteurs présentent un intérêt particulier pour la préparation de milieux de culture permettant une forte production de collagène par les fibroblastes en utilisant une faible dose de palmitate d'ascorbyle.

## PALMITATE D'HYDROLYSAT DE COLLAGENE

Des peptides de collagène sous forme de palmitate ont été utilisés pour stimuler la synthèse des collagènes de type I et III de même que la synthèse de la collagénase interstitielle (MMP-1).

On a pu observer que l'hydrolysat de collagène incorporé dans les vecteurs vésiculaires selon l'invention est moins toxique que sous forme soluble. En effet, une cytotoxicité est détectée à partir d'une dose de 20 µg / ml pour la molécule soluble Tableau 2).

### TABLEAU 2
### Analyse de la prolifération des fibroblastes traités avec le Palmitate d'hydrolysat de collagène

| Forme | µg / ml | | | | | |
|---|---|---|---|---|---|---|
| | 1 | 5 | 10 | 20 | 40 | 80 |
| **24 heures** | | | | | | |
| Solution | $0,486 \pm 0,026$ | $0,481 \pm 0,009$ | $0,450 \pm 0,009$ | $0,290^a \pm 0,011$ | $0,093^a \pm 0,009$ | $0,037^a \pm 0,004$ |
| Vecteur | $0,468 \pm 0,019$ | $0,476 \pm 0,014$ | $0,478 \pm 0,012$ | $0,487^c \pm 0,013$ | $0,496^c \pm 0,015$ | $0,493^c \pm 0,011$ |
| **48 heures** | | | | | | |
| Solution | $0,488 \pm 0,012$ | $0,474 \pm 0,018$ | $0,470 \pm 0,008$ | $0,138^a \pm 0,017$ | $0,058^a \pm 0,010$ | $0,033^a \pm 0,008$ |
| Vecteur | $0,493 \pm 0,017$ | $0,489 \pm 0,007$ | $0,494 \pm 0,008$ | $0,498^c \pm 0,017$ | $0,492^c \pm 0,013$ | $0,482^c \pm 0,006$ |
| **72 heures** | | | | | | |
| Solution | $0,491 \pm 0,010$ | $0,492 \pm 0,021$ | $0,466 \pm 0,019$ | $0,114^a \pm 0,007$ | $0,043^a \pm 0,003$ | $0,023^a \pm 0,005$ |
| Vecteur | $0,497 \pm 0,017$ | $0,489 \pm 0,016$ | $0,491 \pm 0,011$ | $0,478^c \pm 0,012$ | $0,502^c \pm 0,021$ | $0,479^c \pm 0,009$ |

Les données sont exprimées en densité optique mesurée après traitement sur des cultures contenant $10^4$ cellules par puits

[a] significatif ($p < 0,05$) par rapport aux doses les plus faibles

[c] significatif ($p < 0,05$) par rapport à la molécule sous forme soluble

En ce qui concerne la synthèse du collagène, on a noté qu'à partir d'une dose de 5 µg / ml, l'hydrolysat de collagène stimule de façon significative la synthèse du collagène de type I et de type III. Cet effet est observé en utilisant la molécule incorporée aux vecteurs vésiculaires (tableaux 3 et 4).

## TABLEAU 3

## ANALYSE DE LA SYNTHESE DE COLLAGENE DE TYPE I PAR DES FIBROBLASTES TRAITES AVEC LE PALMITATE D'HYDROLYSAT DE COLLAGENE

| Forme | μg/ml | | | | | |
|---|---|---|---|---|---|---|
| | 1 | 5 | 10 | 20 | 40 | 80 |
| **24 heures** | | | | | | |
| Témoin | $274 \pm 34$ | $253 \pm 22$ | $228 \pm 8$ | $187^a \pm 10$ | $160^a \pm 15$ | $135^a \pm 10$ |
| Solution | $274 \pm 28$ | $211^a \pm 12$ | $203^a \pm 13$ | $63^{a,b} \pm 2$ | $49^{a,b} \pm 13$ | $30^{a,b} \pm 3$ |
| Vecteur | $239 \pm 23$ | $297 \pm 28$ | $213 \pm 20$ | $229^c \pm 21$ | $225^{b,c} \pm 27$ | $221^{b,c} \pm 5$ |
| **48 heures** | | | | | | |
| Témoin | $406 \pm 37$ | $360 \pm 11$ | $299^a \pm 27$ | $258^a \pm 4$ | $207^a \pm 5$ | $183^a \pm 5$ |
| Solution | $454 \pm 65$ | $358 \pm 13$ | $272^a \pm 11$ | $80^{a,b} \pm 5$ | $31^{a,b} \pm 8$ | $41^{a,b} \pm 4$ |
| Vecteur | $536^b \pm 32$ | $502^{b,c} \pm 26$ | $470^{b,c} \pm 48$ | $357^{a,b,c} \pm 16$ | $330^{a,b,c} \pm 16$ | $304^{a,b,c} \pm 24$ |
| **72 heures** | | | | | | |
| Témoin | $454 \pm 37$ | $383 \pm 25$ | $249^a \pm 40$ | $246^a \pm 3$ | $232^a \pm 8$ | $219^a \pm 5$ |
| Solution | $456 \pm 29$ | $353^a \pm 9$ | $302^a \pm 5$ | $97^{a,b} \pm 5$ | $19^{a,b} \pm 5$ | $14^{a,b} \pm 3$ |
| Vecteur | $601 \pm 128$ | $577^{b,c} \pm 41$ | $457^{b,c} \pm 13$ | $352^{a,b,c} \pm 20$ | $360^{a,b,c} \pm 23$ | $325^{a,b,c} \pm 10$ |

Les données sont exprimées en ng/ml

[a] significatif (p > 0,05) par rapport aux doses plus faibles

[b] significatif (p > 0,05) par rapport au témoin

[c] significatif (p > 0,05) par rapport à la molécule sous forme soluble

## TABLEAU 4

### Analyse de la synthèse de collagène type III par des fibroblastes traités avec le palmitate d'hydrolysat de collagène

| Forme | µg/ml | | | | | |
|---|---|---|---|---|---|---|
| | 1 | 5 | 10 | 20 | 40 | 80 |
| **24 heures** | | | | | | |
| Témoin | $258 \pm 57$ | $253 \pm 43$ | $183 \pm 18$ | $183 \pm 30$ | $127^a \pm 22$ | $49^a \pm 3$ |
| Solution | $318 \pm 67$ | $292 \pm 25$ | $277^b \pm 17$ | $25^{a,b} \pm 1$ | $23^{a,b} \pm 2$ | $19^{a,b} \pm 1$ |
| Vecteur | $309 \pm 45$ | $311 \pm 12$ | $261^c \pm 9$ | $255^c \pm 34$ | $211^{b,c} \pm 29$ | $207^{a,b,c} \pm 20$ |
| **48 heures** | | | | | | |
| Témoin | $498 \pm 74$ | $260 \pm 15$ | $206^a \pm 30$ | $210^a \pm 10$ | $183^a \pm 21$ | $106^a \pm 6$ |
| Solution | $744 \pm 123$ | $492^{a,b} \pm 15$ | $312^{a,b} \pm 23$ | $27^{a,b} \pm 1$ | $20^{a,b} \pm 3$ | $25^{a,b} \pm 2$ |
| Vecteur | $780^b \pm 76$ | $677^{b,c} \pm 126$ | $665^{b,c} \pm 120$ | $518^{a,b,c} \pm 29$ | $382^{a,b,c} \pm 48$ | $309^{a,b,c} \pm 26$ |
| **72 heures** | | | | | | |
| Témoin | $507 \pm 30$ | $268^a \pm 8$ | $182^a \pm 37$ | $163^a \pm 12$ | $173^a \pm 29$ | $128^a \pm 1$ |
| Solution | $569 \pm 61$ | $446^b \pm 39$ | $352^{a,b} \pm 44$ | $31^{a,b} \pm 3$ | $18^{a,b} \pm 1$ | $15^{a,b} \pm 2$ |
| Vecteur | $680^b \pm 39$ | $793^{b,c} \pm 77$ | $480^{a,b,c} \pm 47$ | $415^{a,b,c} \pm 33$ | $352^{a,b,c} \pm 20$ | $342^{a,b,c} \pm 22$ |

Les données sont exprimées en ng/ml

[a] significatif ($p > 0,05$) par rapport aux doses plus faibles

[b] significatif ($p > 0,05$) par rapport au témoin

[c] significatif ($p > 0,05$) par rapport à la molécule sous forme soluble

La synthèse du collagène de type III n'est stimulée qu'avec une dose de 10 µg/ ml et uniquement lorsque l'hydrolysat de collagène est incorporé aux vecteurs vésiculaires infracellulaires selon l'invention.

Ce même traitement stimule de manière significative la synthèse de collagénase interstitielle (MMP-1) que ce soit sous forme soluble ou sous forme vectoriale. Il faut cependant noter une stimulation significativement plus forte lorsque les molécules sont incorporées dans les vecteurs vésiculaires (tableau 5).

## TABLEAU 5

### Dosage de l'activité de la collagénase interstitielle (MMP-1) produite par des fibroblastes traités avec le Palmitate d'hydrolysat de collagène

| Forme | µg/ml | | |
|---|---|---|---|
| | 1 | 5 | 10 |
| Solution | $1551^a \pm 110$ | $1158^a \pm 152$ | $1144^a \pm 41$ |
| Vecteur | $2372^{a,b} \pm 328$ | $2463^{a,b} \pm 167$ | $2050^{a,b} \pm 87$ |

Témoin : $438 \pm 82$

Ces données sont exprimées en mU/ml des surnageants des cultures avec $4.10^5$ cellules et mesurées après 48 heures de traitement.

[a] significatif ($p < 0,05$) par rapport au témoin

[b] significatif ($p < 0,05$) par rapport à la molécule sous forme soluble.

Les résultats ci-dessus laissent apparaître tout l'intérêt de la forme vectoriale pour l'obtention de produits cosmétiques permettant la simulation de la synthèse collagénique et du renouvellement de la matrice extra-cellulaire du derme (synthèse de collagène et synthèse de collagénase) et notamment le raffermissement de la peau d'individus âgés.

Cette forme vectoriale présente également un intérêt particulier pour l'obtention de compositions médicamenteuses favorisant un renouvellement de la matrice extracellulaire par des activités concomitantes de synthèse de molécules nouvelles (collagène) et de dégradation de la matrice pré-existante (collagénase). Les molécules incorporées dans les vecteurs sont moins toxiques.Ces médicaments peuvent trouver une application dans le cas des cicatrices hypertrophiques ou kéloïdes qui sont à l'heure actuelle traitées avec des cytokines (Granstein et coll. The Jal of Investigative Dermatology Supl. déc. 1990 - p. 75 S-80 S) (Kovacs - Immunology Today 1 - 1991).

Enfin les résultats ci-dessus laissent apparaître tout l'intérêt de la forme vectoriale pour la préparation de milieux de culture destinés à stimuler la synthèse de collagène de type I et de son enzyme de dégradation (collagénase interstitielle) par les fibroblastes.

De plus les molécules incorporées dans les vecteurs vésiculaires infracellulaires sont moins toxiques.

## PALMITATE D'HYDROXYPROLINE

Le traitement de cultures de fibroblastes avec le palmitate d'hydroxyproline montre que la forme soluble est cytotoxique à partir de 20 µg / ml.

Cependant son incorporation dans les vecteurs élimine cet effet cytotoxique même à une dose de 80 µg / ml (tableau 6).

## TABLEAU 6

## Analyse de la prolifération des fibroblastes traités avec le Palmitate d'hydroxyproline

| Forme | µg / ml | | | | | |
|---|---|---|---|---|---|---|
| | 1 | 5 | 10 | 20 | 40 | 80 |
| **24 heures** | | | | | | |
| Solution | 0,493 ± 0,029 | 0,492 ± 0,016 | 0,502 ± 0,019 | 0,457 ± 0,022 | 0,147$^a$ ± 0,019 | 0,060$^a$ ±0,022 |
| Vecteur | 0,483 ± 0,026 | 0,479 ± 0,029 | 0,485 ± 0,033 | 0,502 ±0,037 | 0,487$^c$± 0,019 | 0,485$^c$± 0,023 |
| **48 heures** | | | | | | |
| Solution | 0,494 ± 0,012 | 0,513 ± 0,025 | 0,511 ± 0,020 | 0,471 ± 0,028 | 0,118$^a$ ±0,018 | 0,060$^a$ ± 0,016 |
| Vecteur | 0,493 ±0,021 | 0,495 ± 0,025 | 0,487 ± 0,035 | 0,519 ± 0,024 | 0,545$^c$ ±0,021 | 0,556$^{a,c}$±0,022 |
| **72 heures** | | | | | | |
| Solution | 0,490 ± 0,019 | 0,500 ± 0,028 | 0,496 ± 0,017 | 0,414$^a$ ± 0,010 | 0,063$^a$ ± 0,021 | 0,033$^a$ ± 0,012 |
| Vecteur | 0,505 ± 0,018 | 0,515 ± 0,017 | 0,506 ± 0,020 | 0,498$^c$± 0,015 | 0,495$^c$ ± 0,018 | 0,520$^c$ ± 0,022 |

Les données sont exprimées en densité optique mesurée après traitement sur des cultures contenant $10^4$ cellules par puit

$^a$ significatif (p<0,05) par rapport aux doses plus faibles

$^c$ significatif (p < 0,05) par rapport à la molécule sous forme soluble

La synthèse de collagène type I et III est stimulée de façon significative par la forme vectoriale de la molécule à une dose de 10 µg / ml (tableaux 7 et 8).

## TABLEAU 7

### ANALYSE DE LA SYNTHESE DE COLLAGENE TYPE I PAR DES FIBROBLASTES TRAITES AFEC LE PALMITATE D'HYDROXYPROLINE

| Forme | μg/ml | | | | | |
|---|---|---|---|---|---|---|
| | 1 | 5 | 10 | 20 | 40 | 80 |
| **24 heures** | | | | | | |
| Témoin | 246 ± 46 | 275 ± 22 | 292 ± 3 | 259 ± 8 | 221 ± 11 | $145^{a} \pm 12$ |
| Solution | 268 ± 18 | 232 ± 10 | $166^{a,b} \pm 15$ | $128^{a,b} \pm 4$ | $61^{a,b} \pm 8$ | $41^{a,b} \pm 2$ |
| Vecteur | 266 ± 10 | $288^{c} \pm 14$ | $253^{c} \pm 11$ | $251^{c} \pm 16$ | $184^{a,c} \pm 31$ | $164^{a,c} \pm 7$ |
| **48 heures** | | | | | | |
| Témoin | 487 ± 67 | 518 ± 0 | 449 ± 57 | 392 ± 22 | $345^{a} \pm 18$ | $298^{a} \pm 15$ |
| Solution | 519 ± 37 | $360^{a,b} \pm 42$ | $260^{a,b} \pm 7$ | $227^{a,b} \pm 11$ | $96^{a,b} \pm 6$ | $49^{a,b} \pm 10$ |
| Vecteur | 528 ± 30 | $605^{c} \pm 43$ | $423^{c} \pm 69$ | $421^{a,c} \pm 9$ | $422^{a,b,c} \pm 26$ | $401^{a,b,c} \pm 18$ |
| **72 heures** | | | | | | |
| Témoin | 431 ± 8 | 446 ± 15 | 446 ± 52 | 499 ± 39 | $368^{a} \pm 18$ | $297^{a} \pm 5$ |
| Solution | 513 ± 77 | 470 ± 5 | $281^{a,b} \pm 15$ | $223^{a,b} \pm 29$ | $73^{a,b} \pm 10$ | $49^{a,b} \pm 3$ |
| Vecteur | 483 ± 46 | 516 ± 78 | $524^{c} \pm 17$ | $526^{c} \pm 90$ | $387^{a,c} \pm 10$ | $354^{a,b,c} \pm 20$ |

Les données sont exprimées en ng/ml

[a] significatif ($p > 0{,}05$) par rapport aux doses plus faibles

[b] significatif ($p > 0{,}05$) par rapport au témoin

[c] significatif ($p > 0{,}05$) par rapport à la molécule sous forme soluble

## TABLEAU 8

### Analyse de la synthèse de collagène type III par des fibroblastes traités avec le palmitate d'hydroxyproline

| Forme | µg/ml | | | | | |
|---|---|---|---|---|---|---|
| | 1 | 5 | 10 | 20 | 40 | 80 |
| **24 heures** | | | | | | |
| Témoin | 239±33 | 221 ± 31 | 224 ± 17 | 211 ± 14 | $137^a \pm 9$ | $81^a \pm 10$ |
| Solution | 295 ± 24 | 281 ± 16 | $154^{a,b} \pm 13$ | $105^{a,b} \pm 6$ | $25^{a,b} \pm 2$ | $24^{a,b} \pm 4$ |
| Vecteur | 272 ± 21 | $292^b \pm 2$ | $290^{b,c} \pm 16$ | $313^{b,c} \pm 22$ | $215^{a,b,c} \pm 14$ | $119^{a,b,c} \pm 10$ |
| **48 heures** | | | | | | |
| Témoin | 624 ± 83 | 528 ± 47 | $376^a \pm 36$ | $385^a \pm 14$ | $369^a \pm 23$ | $310^a \pm 14$ |
| Solution | 588 ± 69 | 519 ± 20 | $330^a \pm 35$ | $253^{a,b} \pm 18$ | $36^{a,b} \pm 5$ | $18^{a,b} \pm 3$ |
| Vecteur | 699 ± 117 | $702^{b,c} \pm 14$ | $509^{b,c} \pm 65$ | $525^{b,c} \pm 14$ | $532^{b,c} \pm 91$ | $494^{b,c} \pm 20$ |
| **72 heures** | | | | | | |
| Témoin | 452 ± 41 | 450 ± 46 | 405 ± 49 | 385 ± 18 | $313^a \pm 29$ | $280^a \pm 18$ |
| Solution | 628 ± 161 | 548 ± 24 | $301^a \pm 23$ | $240^{a,b} \pm 11$ | $29^{a,b} \pm 1$ | $24^{a,b} \pm 1$ |
| Vecteur | 519 ± 108 | $558^b \pm 7$ | $616^{b,c} \pm 91$ | $637^{b,c} \pm 38$ | $444^{b,c} \pm 27$ | $419^{b,c} \pm 26$ |

Les données sont exprimées en ng/ml

[a] significatif (p > 0,05) par rapport aux doses plus faibles

[b] significatif (p > 0,05) par rapport au témoin

[c] significatif (p > 0,05) par rapport à la molécule sous forme soluble

L'activité collagénolytique des fibroblastes est modulée par l'utilisation du palmitate d'hydroxyproline et ses effets sont en relation avec la forme d'utilisation : vectoriale ou soluble. La forme soluble stimule de manière significative la synthèse de la collagénase même avec une dose de 1 µg / ml (tableau 9). Par contre l'utilisation de la forme vectoriale du palmitate d'hydroxyproline réduit progressivement, de façon dépendante, la synthèse de collagénase.

## TABLEAU 9

### Dosage de l'activité de la collagénase interstitielle (MMP-1) produite par des fibroblastes traités avec le Palmitate d'hydroxyproline

| Forme | µg/ml | | |
|---|---|---|---|
| | 1 | 5 | 10 |
| Solution | 1273[a]± 154 | 1934[a] ± 49 | 2189[a] ± 118 |
| Vecteur | 1648[a,b]± 166 | 1090[a,b]± 66 | 604[a] ± 101 |

Témoin : 438 ± 82

Ces données sont exprimées en mU/ml des surnageants des cultures avec $4.10^5$ cellules et mesurées après 48 heures de traitement.

[a] significatif (p < 0,05) par rapport au témoin

[b] significatif (p < 0,05) par rapport à la molécule sous forme soluble.

La forme vectoriale permet donc l'obtention de produits cosmétiques visant à stimuler la synthèse de collagène afin d'obtenir un meilleur raffermissement de la peau.

Elle permet également l'obtention de compositions médicamenteuses susceptibles de favoriser le renouvellement de la matrice extracellulaire par un effet stimulant la synthèse de collagène par les fibroblastes.

L'incorporation de palmitate d'ascorbyle dans les vecteurs permet une meilleure efficacité à faibles doses.

Les compositions médicamenteuses selon l'invention trouvent, entre autres, des applications à l'augmentation de la synthèse des collagénases en vue de résorber les tissus fibreux denses en collagène I ou les chéloïdes, ainsi qu'à l'orientation de la synthèse du collagène vers une structure particulière telle que les collagènes I et III.

La forme vectoriale permet enfin de préparer des milieux de culture avec le palmitate d'hydroxyproline incorporé dans les vecteurs vésiculaires infracellulaires selon l'invention pour stimuler la synthèse de collagène sans stimuler la synthèse de collagénase. Les milieux de culture selon l'invention trouvent en particulier des applications intéressantes dans la production de peptides de la matrice extracellulaire et la production de peptides à activité enzymatique.

Le tableau 10 ci-après résume les résultats obtenus.

TABLEAU 10

| | Palmitate d'hydrolysat de collagène | | Palmitate d'hydroxyproline | | Palmitate d'ascorbyl | | Hydroxyproline | |
|---|---|---|---|---|---|---|---|---|
| Temps : 48 h | | | | | | | | |
| DOSES | 10 µg/ml | | 40 µg/ml | | 25 µg/ml | | 10 µg/ml | |
| Forme | libre | encaps. | libre | encaps. | libre | encaps. | libre | encaps. |
| Prolifération | ÷10 | = | ÷3 | = | + | = | = | x 1,5 |
| Cytotoxicité Test MTT | oui | non | oui | non | non | non | non | non |
| Synthèse collag. I | ÷2 | ≃ | ÷4 | = | x 15 | x 20 | x 4 | x 5 |
| Synthèse collag. III | = | ≃ | ÷ 10 | x 2 | x 30 | x 50 | x 2 | x 3 |
| Collagénase | x 6 | x 12 | = | ÷ 2 | = | = | = | = |
| CONCLUSION à 4.10⁵cell. | modulants sélectifs de la collagènase | | inhibit. des col-lagènes. | Modu-lant sé-lectif collag. III | Stimulants non sélectifs | | Stimulant sélectif du collagène I | |

Ce tableau montre bien la capacité qu'ont les vecteurs selon l'invention à moduler l'activité des fibroblastes lorsqu'ils portent à leur surface certaines fonctions hydrophiles fixées sur ledit vecteur par un ester ou une amide d'acide gras.

Cette fonction hydrophile est soit un acide aminé, soit un polypeptide (succession d'acides aminés), soit de l'acide ascorbique, la fonction lipophile étant soit une chaîne alkyle pouvant être partiellement insaturée soit un radical aromatique (p.ex. palmitate d'hydroxyproline, oléate d'ascorbyle, acide hippurique : benzoate de glycine).

La modulation de la synthèse des collagénases par l'encapsulation des esters gras de l'hydrolysat de collagène est appréciable pour la forme encapsulée dans les vecteurs selon l'invention, car la forme libre est cytotoxique ; on peut parler de modulation parce que le nombre des cellules reste constant.

De même on peut apprécier la modulation de la synthèse du collagène III par les esters gras ou aromatiques de l'hydroxyproline, la forme libre étant cytotoxique et diminuant la synthèse des collagènes I et III alors que la forme encapsulée p. ex. à 40 µg /ml multiplie par 2 la synthèse du collagène III sans affecter la prolifération des cellules.

**Revendications**

1 - Vecteurs vésiculaires infracellulaires comportant des ligands métaboliques aptes à s'exprimer à l'intérieur et à l'extérieur de leur structure, caractérisés en ce qu'ils sont des vecteurs infracellulaires d'affinité spécifique du fibroblaste dont la composition membranaire, modifiée par introduction de ligands métaboliques, entraîne une modulation de l'activité de synthèse de la cellule ciblée.

2 - Vecteurs vésiculaires selon la revendication 1, caractérisés en ce qu'ils sont choisis parmi les vésicules, capsules ou sphères artificielles d'un diamètre qui peut aller de 30 à 1000 nanomètres, mis en oeuvre pour incorporer une substance active soit dans un milieu aqueux remplissant le volume intérieur du vecteur soit dans la paroi même dudit vecteur puis pour transporter cette substance active vers un site où elle sera libérée et utilisée à différentes fins.

3 - Vecteurs vésiculaires selon la revendication 1 ou la revendication 2, caractérisés en ce que les actifs sont encapsulés à des concentrations variant de 0,01 % à 2 % ( $10^{-5}$ - $10^{-2}$) par rapport à la phase lipidique totale.

**4 -** Vecteurs selon l'une quelconque des revendications 1 à 3, caractérisés en ce que les ligands métaboliques sont choisis parmi les initiateurs et les modulateurs de la synthèse cellulaire dotés de fonction ou radical lipophile par acylation.

**5 -** Vecteurs selon l'une quelconque des revendications 1 à 3, caractérisés en ce que les initiateurs de la synthèse cellulaire sont choisis parmi les esters gras de l'acide ascorbique et plus spécialement son palmitate.

**6 -** Vecteurs selon l'une quelconque des revendications 1 à 3, caractérisés en ce que le modulateur de la collagénase est le palmitate d'hydrolysat de collagène.

**7 -** Vecteurs selon l'une quelconque des revendications 1 à 3, caractérisés en ce que les modulateurs de la synthèse cellulaire sont choisis les esters gras de l'hydroxyproline et plus spécialement son palmitate.

**8 -** Application des vecteurs vésiculaires selon l'une quelconque des revendications 1 à 7 dans les compositions cosmétiques destinées au raffermissement du tissu cutané par augmentation de la synthèse du collagène I ou III.

**9 -** Application des vecteurs vésiculaires selon l'une quelconque des revendications 1 à 7 dans les compositions cosmétiques destinées au traitement des petites cicatrices indurées et des ridules par aumgentation de la synthèse de la collagénase.

**10 -** Application des vecteurs vésiculaires selon l'une quelconque des revendications 1 à 7 dans les compositions cosmétiques destinées à provoquer un renforcement de la couche épithéliale par augmentation de la matrice extra-cellulaire.

**11.** Application des vecteurs vésiculaires selon l'une quelconque des revendications 1 à 7 dans les compositions médicamenteuses destinées à l'augmentation de la synthèse des collagénases en vue de résorber les tissus fibreux denses en collagène I ou les chéloïdes.

**12 -** Application des vecteurs vésiculaires selon l'une quelconque des revendications 1 à 7 dans les compositions médicamenteuses destinées à l'orientation de la synthèse du collagène vers une structure particulière telle que les collagènes I et III.

**13 -** Application des vecteurs vésiculaires selon l'une quelconque des revendications 1 à 7 à la réalisation de milieux de culture pour la production de peptides de la matrice extracellulaire.

**14. -** Application des vecteurs vésiculaires selon l'une quelconque des revendications 1 à 7 à la réalisation de milieux de culture pour la production de peptides à activité enzymatique.

EP 0 537 092 A1

**Office européen des brevets**

**RAPPORT PARTIEL DE RECHERCHE EUROPEENNE**
qui selon la règle 45 de la Convention sur le brevet européen est consideré, aux fins de la procédure ultérieure comme le rapport de la recherche européenne

Numero de la demande

EP 92 42 0355

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl. 5) |
|---|---|---|---|
| X | FR-A-2 540 381 (PARFUMS CHRISTIAN DIOR) * revendications * | 1-4,8-14 | A61K9/127 A61K7/00 C12N5/00 |
| X | EP-A-0 160 286 (TERUMO CORPORATION) * page 5, ligne 25 - page 6, ligne 6; revendications * | 1-5 | |
| X | WO-A-8 706 460 (L'OREAL) * revendications * | 1-4,6-12 | |
| X | EP-A-0 424 282 (ERPHAR) * revendication 5 * | 1-4,7-10 | |

**DOMAINES TECHNIQUES RECHERCHES (Int. Cl. 5)**

C12N
A61K
A61K
C07K

### RECHERCHE INCOMPLETE

La division de la recherche estime que la présente demande de brevet européen n'est pas conforme aux dispositions de la Convention sur le brevet européen au point qu'une recherche significative sur l'état de la technique ne peut être effectuée au regard d'une partie des revendications.
Revendications ayant fait l'objet de recherches complètes:
Revendications ayant fait l'objet de recherches incomplètes:
Revendications n'ayant pas fait l'objet de recherches:
Raison pour la limitation de la recherche:

voir feuille supplémentaire C

| Lieu de la recherche | Date d'achèvement de la recherche | Examinateur |
|---|---|---|
| LA HAYE | 19 JANVIER 1993 | NOOIJ F.J.M. |

CATEGORIE DES DOCUMENTS CITES
X : particulièrement pertinent à lui seul
Y : particulièrement pertinent en combinaison avec un autre document de la même catégorie
A : arrière-plan technologique
O : divulgation non-écrite
P : document intercalaire
T : théorie ou principe à la base de l'invention
E : document de brevet antérieur, mais publié à la date de dépôt ou après cette date
D : cité dans la demande
L : cité pour d'autres raisons
& : membre de la même famille, document correspondant

16

Feuille C

Remarque:

Bien que les revendications 11 et 12 concernent une méthode de traitement du corps humain/animal (Article 52(4) CEB), la recherche a été effectuée et basée sur les effects inputés au produit/ à la composition.